# EUROPEAN PATENT APPLICATION

(11) **EP 3 135 242 A1**
(43) Date of publication of application: **01.03.2017**
(21) Application number: 15782740.3
(22) Date of filing: 24.04.2015
(51) Int. Cl.: A61C 13/00, A61L 27/06, A61L 27/30, A61F 2/28, C23C 14/16, C23C 14/35

(54) **BIOCOMPATIBLE IMPLANTS MADE OF NANOSTRUCTURED TITANIUM WITH ANTIBACTERIAL PROPERTIES**

(30) Priority: 25.04.2014 ES 201430616
(71) Applicant: Consejo Superior de Investigaciones Cientificas (CSIC), 28006 Madrid (ES); Universidad Complutense De Madrid, 28015 Madrid (ES)
(72) Inventor: GARCÍA MARTÍN, José Miguel, E-28760 Tres Cantos (Madrid) (ES); PALMERO ACEBEDO, Alberto, E-41092 Sevilla (ES); ÁLVAREZ MOLINA, Rafael, E-41092 Sevilla (ES); VALLET-REGÍ, María, E-28015 Madrid (ES); ARCOS NAVARRETE, Daniel, E-28015 Madrid (ES); IZQUIERDO BARBA, Isabel, E-28015 Madrid (ES)
(74) Representative: Pons Ariño, Angel
(86) International application number: PCT/ES2015/070345
(87) International publication number: WO 2015/162329

(57) **Abstract**

The invention relates to a novel implant based on titanium, consisting of a titanium coating formed over biomaterials, applicable in bone implantology. The nanotopographical characteristics of said implants inhibit bacterial adhesion and formation of bacterial biofilm on the surface, the implants simultaneously exhibiting suitable properties for the adhesion, extension, and proliferation of the bone-forming cells. The invention also relates to a method for producing the implant by means of oblique incidence techniques and to the use thereof in bone implantology..

## Description

### TECHNICAL SECTOR AND OBJECT OF THE INVENTION

The invention is framed within coating materials and adhesives for medical applications, medical engineering or sanitary engineering in the broadest sense of the words. The invention is also framed within the field of nanomaterials with medical applications.

The object of the invention is an implant composed of a titanium coating deposited on a substrate that comprises a biomaterial for surgical applications, which has osseointegrative and bacterial biofilm formation inhibitory properties, as well as methods for manufacturing the implants by means of oblique-incidence techniques, preferably by means of cathode sputtering, and the use thereof in osseous implantology.

### PRIOR ART

Titanium and its alloys are widely used in osseous implantology because of their exceptional biocompatibility, excellent mechanical properties and corrosion resistance. This high corrosion resistance primarily lies on the rapid formation of a titanium oxide layer on the surface thereof, known as dry corrosion or passivation of titanium. The formation of titanium oxide takes place spontaneously by the oxidation of the metal surface upon coming in contact with atmospheric oxygen. The passivation layer provides titanium-based implants with excellent anti-corrosion properties and osseointegrative properties.

Most metallic orthopaedic implant infections are caused by bacteria of the *Staphylococcus* type. Amongst them, *Staphylococcus aureus* (*S. aureus*) represents the primary pathogenic species in the case of metallic biomaterials such as stainless steel, CrCo, Ti and the alloys thereof. When the bacteria adhere to the surface of the implant, they secrete an extracellular polymeric matrix called biofilm, which provides them with high resistance to antibiotics (F. Götz et al., Molecular Microbiology 43, 1367, 2002*).* The formation of this biofilm almost always forces removal of the prosthesis, since, otherwise, the implant infection may lead to chronic infections and, in extreme cases, even to death of the patient due to septicaemia.

Currently, there are several strategies that attempt to prevent the problem of implant infection, for example, implanting polymethylmethacrylate beads loaded with broad-spectrum antibiotics. This strategy has the limitation that the beads require a second surgery in order to be removed. On the other hand, once the implant has become infected, the strategy to be followed is the systemic administration of high doses of antibiotics, with the consequent toxicity problems. In any event, when bacteria manage to form a bacterial biofilm, they become very resistant to treatment with systemic antibiotics and the implant must be removed in order to prevent chronic infections and septicaemia. For all these reasons, addressing the problem of infection from the first stage, by preventing bacterial adhesion, entails a significant advance in the prevention of bone implant infections.

In regards to orthopaedic and dental implants, the formation of nanostructures by means of various techniques (anodisation, vapour deposition, etc.) designed to favour the adhesion of bone-forming cells (osteoblasts), as well as to improve the behaviour thereof once they have become adhered (differentiation, formation of an extracellular matrix, etc.), has been disclosed in the field. Thus, for example, nanostructured implant coatings have been synthesised by means of various techniques and with different forms; examples include TiO₂ nanotubes manufactured by means of anodisation techniques (M. Ma, et al., J. Biomedical Material Research Part A 100, 278, 2012), as well as nanostructured hydroxyapatites obtained by means of the hydrolysis of solid precursors, metallic alloys obtained by means of low-temperature powder metallurgy, titanium nanostructures obtained by means of chemical nanotopography and titanium nanostructures obtained by means of superficial oxidation.

On the other hand, Ti nanostructures able to inhibit bacterial adhesion have also been prepared (D. Campoccia, Biomaterials 34, 8533, 2013; K. Anselme, Acta Biomaterialia 6, 3824, 2010). For example, in some cases, the TiO₂ present on the surface has demonstrated a certain bactericidal capacity after being irradiated with UV light.

There are several works that describe nanotopographies that preserve their behaviour with respect to osteoblasts, but simultaneously inhibit bacterial colonisation (Colon et al., J Biomedical Materials Research A 78, 595, 2006, and Ploux L. et al., Langmuir 25, 8161, 2009). In both cases, the studies were conducted on materials without clinical application thus far. In M. Kazemzadeh-Narbat et al., Biomaterials 34 5969 2013, both effects are achieved (biocompatibility with osteoblasts and antimicrobial activity) by using titanium obtained by means of anodisation processes, but they are based on the incorporation of drugs on the layers grown, for which reason their antimicrobial activity is not caused by the nanostructure grown, but by the incorporated medicament.

One technique that is widely used in microelectronics and which allows for the formation of nanostructures with a large variety of properties, such as topographies, compositions, etc., is the so-called cathode sputtering technique (P.J. Kelly et al., Vacuum 56, 159, 2000). This technique has been well-known for decades, since it grows thin sheets that are very compact and have low roughness. In it, a solid block, also called target, of a given material (in this case titanium) is placed inside a reactor or vacuum chamber containing an inert gas (in general, argon gas is used). Upon injecting electromagnetic power through the target by means of an excitation source, a gaseous plasma rich in energetic ions is generated, which sputters the surface of the target, and atoms are emitted, preferably in a direction perpendicular to the target, with kinetic energies of the order of 10 eV. When they reach a surface in the interior of the reactor parallel to the target, called substrate, these atoms progressively accumulate and agglomerate on the surface, thereby generating a thin film. Depending on the operating pressure in the chamber, resulting from the inert gas introduced, the energy with which said atoms reach the surface may be controlled. At high pressures (above 1 Pa under standard conditions), there are numerous collisions, for which reason the atoms reach the substrate with low energy (tenths or hundredths of eV). However, at low pressures, the input energy is very similar to the output energy of the target (ballistic regime), which generates highly compact thin films.

Cathode sputtering is a process that is very widely used in the formation of thin films on materials; in fact, cathode sputtering is used industrially in multiple applications: manufacturing of hard drives, mirrors, grocery bag inner coatings, etc. Contrary to techniques that involve some type of chemical reaction, such as chemical synthesis, anodisation, photolithography, etc., cathode sputtering is a vacuum technique; consequently, no environmentally aggressive residues are generated and, moreover, it is efficient from an energetic standpoint, since it allows for manufacturing at low temperatures (room temperature). Contrary to other physical vacuum techniques, such as thermal evaporation or electron-gun-assisted evaporation, cathode sputtering is widely used in industry and allows for the growth of the nanostructured material on large surfaces with different morphologies.

In recent years, cathode sputtering is also being used in oblique-angle geometries; this is the technique known as glancing-angle cathode sputtering, or GLAD sputtering, in the literature (J. C. Sit et al., Journal of Materials Research 14 (4), 1197, 1999). In this case, following the generation of plasma in the chamber, the substrate whereon the atoms accumulate is no longer placed parallel to the target, but forms a tilt angle greater than 60° with respect to it, the so-called GLAD angle, which causes the atoms to reach the substrate with oblique incidence. This configuration induces shadowatomistic shadowing processes on the surface of the growing thin layer that generate tilted structures.

Thus far, no coatings have been prepared for bone implants with a biomaterial that may be used to coat implants or prostheses, formed by Ti nanotopographies which simultaneously allow for the adhesion and proliferation of osteoblasts whilst inhibiting bacterial colonisation. These studies have always been conducted separately with other techniques and most of them have used bacteria that do not have a significant incidence on prosthesis infections.

### DESCRIPTION OF THE INVENTION

A first aspect of the invention is an implant which has a titanium coating deposited on a substrate, characterised in that:
- the substrate comprises a biomaterial with a root mean square roughness lower than 5 nm on a surface area of 4 µm²,
- the coating has a purity greater than 95% and comprises nanostructured titanium formed by metallic titanium and a titanium oxide layer,
- the nanostructured titanium has a nanocolumnar shape, where the diameter of the nanocolumns ranges between 30 and 100 nm, the height ranges between 100 and 300 nanometres, and the space between the nanocolumns ranges between 50 and 150 nanometres, with a nanocolumn tilt angle with respect to the vertical of the substrate ranging between 0° and 30°.

The substrate biomaterial may have at least one of the following materials:
- commercially-pure medical-grade titanium with a purity greater than 99%, for periodontal implants,
- medical-grade metallic alloys, such as CrCo, stainless steel and Ti6Al4V, for orthopaedic, cranial and maxillofacial applications.

The substrate biomaterial may be shaped into structures that comprise discs, bolts, nails, rods, osteosynthesis plates and other fracture fixation devices, generally manufactured with stainless steel.

Another aspect of the invention is the process for obtaining the implant, which comprises depositing the coating on the substrate by means of glancing-angle techniques (GLAD), preferably in a cathode sputtering system.

The cathode sputtering may be of the magnetron type.

The process may comprise the following steps:
a) introduction of the substrate in the cathode sputtering system chamber,
b) closing of the chamber and creation of a vacuum,
c) introduction of gas into the chamber,
d) electromagnetic excitation of the gas particles present in the chamber by means of a source,
e) collision of the particles present in the chamber against a titanium target,
f) deposition of the material detached from the target on the substrate,
characterised in that the product of multiplying the operating pressure (P_{g}) by the target-substrate distance (L) fulfils the ballistic regime condition for the sputtering of Ti, given by p_{g}L < 12 Pa cm, and the substrate forms a tilt angle greater than 60° with respect to the target.

In a particular case, the vacuum obtained is less than 10⁻⁴ Pa, and the chamber fulfils the condition that the L/d quotient, where d is the diameter of the target and L is the target-substrate distance, be greater than 3.5.

The third aspect of the invention is the use of the implant in osseous implantology.

Another aspect of the invention is a treatment method for humans or animal which comprises the following steps:
- inserting the implant in a human or animal body by means of surgery, preferably by means of orthopaedic, cranial, dental and/or maxillofacial surgery.

In a particular embodiment, the implant may be for temporary use or for permanent use.

### DETAILED DESCRIPTION OF THE INVENTION

The invention is based on the fact that the inventors have obtained a new type of titanium coatings for metallic biomaterials of surgical interest which present a selective behaviour towards bacteria and osteoblasts. The nanotopographical characteristics of these coatings inhibit bacterial adhesion and the formation of a bacterial biofilm on the surface, whilst simultaneously presenting suitable properties for the adhesion, stretching and proliferation of bone-forming cells.

One object of the invention is an implant that comprises a titanium coating deposited on a substrate, characterised in that:
- the substrate comprises a biomaterial with a root mean square roughness lower than 5 nm on a surface area of 4 µm²,
- the coating has a purity greater than 95% and comprises nanostructured titanium formed by metallic titanium and a titanium oxide layer,
- the nanostructured titanium has a nanocolumnar shape, where the diameter of the nanocolumns ranges between 30 and 100 nm, the height ranges between 100 and 300 nanometres, and the space between the nanocolumns ranges between 50 and 150 nanometres, with a nanocolumn tilt angle with respect to the vertical of the substrate ranging between 0° and 30°.

The substrate is decisive for the formation of the coating, since its purity, material, topography, etc. affect the structural properties of the coating deposited thereon. If a suitable substrate is not used, a coating is not formed with the necessary properties to act as an implant.

In a particular embodiment, the substrate biomaterial comprises at least one of the following materials:
- commercially-pure medical-grade titanium with a purity greater than 99%, for periodontal implants,
- medical-grade metallic alloys, such as CrCo, stainless steel and Ti6Al4V, for orthopaedic, cranial and maxillofacial applications.

These biomaterials are of great importance in the manufacturing of modular articular prostheses. Ti6Al4V alloys are the biomaterial of choice to manufacture the prosthesis component implanted in the medullary cavity of the bone, since they present excellent osseointegration and an elastic modulus of about 110 GPa, which allows for the transfer of the mechanical load from the material to the bone. This value represents one-half with respect to that of stainless steel and CrCo. The lower rigidity of Ti6Al4V prevents protective situations against the load, which are associated with the loss of bone in the region surrounding the implant and subsequent loosening of the prosthesis. On the contrary, for the components that are a part of the friction pair in articular prostheses, the materials of choice are CrCo alloys or stainless steel, due to their better behaviour towards friction.

In a more particular embodiment, the substrate biomaterial is manufactured in the form of implantable devices that comprise discs, bolts, nails, rods and osteosynthesis plates, as well as other fracture fixation devices.

The coating may have additional layers of material; for example, one layer between the coating and the substrate which favours the adhesion of the nanostructured titanium grown thereon.

The surface of the implant is the surface of the coating and is defined by the formation of nanocolumns of nanostructured titanium. This surface inhibits the formation of a bacterial biofilm. Moreover, bacterial adhesion on the coating is much lower than that observed in uncoated biomaterials. Specifically, the microbiological studies performed in this invention with *S*. *aureus* show that the coatings are effective surfaces to inhibit the adhesion of this pathogen. The inhibition of bacterial adhesion prevents the subsequent formation of colonies and the subsequent development of a bacterial biofilm.

Regardless of the type of cell, prokaryotic in the case of bacteria or eukaryotic in the case of osteoblasts, there are a number of common steps in the colonisation of surfaces. Cell adhesion to a substrate is sequentially produced by the cell in different steps: i) recognition of the surface, ii) formation of initial contacts, and their subsequent development into focal points, and iii) cellular expansion and development (S. Faghilhi, et al., Journal of Biomedical Materials Research, Part A 91 656 2009). In this case, the capacity to control the topology of the surface, not only in regards to the size of the nanocolumns, but also to their spacing and surface chemistry, makes it possible to design surfaces that present a selective behaviour on the basis of the differential characteristics of both types of cells.

Osteoblasts are cells that have lateral dimensions ranging between 10 and 50 µm. They have a flexible cell membrane, which allows them to adapt to different nanotopographies. Osteoblasts require the prior adsorption of integrins on the surface of the material in order to adhere thereto. The coatings described in this invention show that the adsorption of integrins is sufficient to facilitate the adhesion, stretching and proliferation of osteoblasts, with the same efficacy as on medical-grade polished Ti6Al4V surfaces. On the contrary, bacteria do not need the prior adsorption of proteins on the surface in order to colonise them. On the other hand, bacteria generally have a characteristic shape and are less deformable. Specifically, *S*. *aureus* presents dimensions of 1 µm in diameter and the bacterial cell wall is much more rigid than eukaryotic cell membranes. These differential characteristics with respect to osteoblasts result in *S*. *aureus* presenting a high sensitivity towards the nanotopography. The roughness and the short distance between the nanocolumns exert a double effect on *S*. *aureus* which limits the adhesion thereof. On the one hand, the bacteria have access to a very limited number of anchor points, since their small size and their rigidity allow them to come in contact only with the upper surface of a limited number of nanocolumns in order to develop adhesion focal points. On the other hand, the air trapped between the nanocolumns exerts a low-wettability effect analogous to that of lotus leaves, which further hinders the adhesion of *S. aureus* and the development of the extracellular polymeric matrix that generates the bacterial biofilm.

Surprisingly, whereas, under the conditions of this work, *S. aureus* develops a biofilm in commercial medical-grade Ti6Al4V substrate samples after several days of culture (3 days in the particular case of a Ti6Al4V substrate), the surface of the implant which in this particular case is the surface of the coating deposited on these same substrates, or nano-Ti6Al4V, shows a selective behaviour depending on the type of cell. Whereas osteoblasts adhere to and proliferate on the surface of nano-Ti6Al4V to the same extent as with Ti6Al4V, the adhesion of *S. aureus* is seriously hindered and the formation of a biofilm is inhibited when it is cultured on the nanostructured surface.

In regards to the behaviour towards osteoblasts, the nanostructural characteristics of nanostructured titanium, such as the high density of the nanocolumns and the short spacing between them (with a mean value of 100 nm), cause them not to modify their behaviour with respect to polished Ti6Al4V surfaces. In this sense, the larger size of osteoblasts, their flexibility and the adhesion of integrins on the titanium oxide passivation layer of the nanocolumns allow for excellent cellular development on these coatings, in a manner analogous to that of medical-grade Ti6Al4V alloys.

Overall, this property of the substrate coatings allows for the osseointegration of the implants, thereby preventing the potential infections that may arise in a relatively short period of time following the implantation, which usually forces the removal and replacement of the prosthesis.

The second object of the invention is a process for obtaining the implant, hereinafter process, which comprises depositing the coating on the substrate using glancing-angle techniques (GLAD), preferably in a cathode sputtering system.

The cathode sputtering system may be of the magnetron type, that is, one that uses, at least, one magnetron, i.e. uses magnets that concentrate the ionisation of the gas in the vicinity of the target; this makes ionisation in the rest of the vacuum chamber more rare and makes it possible to work with lower gas pressures to obtain the coating structure.

In a particular embodiment, the deposition is performed in a cathode sputtering system and comprises the following steps:
a) introduction of the substrate into the cathode sputtering system chamber,
b) closing of the chamber and creation of a vacuum,
c) introduction of gas into the chamber,
d) electromagnetic excitation of the gas particles present in the chamber by means of a source,
e) collision of the particles present in the chamber against a titanium target,
f) deposition of the material detached from the target on the substrate,
characterised in that the product of multiplying the operating pressure (P_{g}) by the target-substrate distance (L) fulfils the ballistic regime condition for the sputtering of Ti, defined by p_{g}L < 12 Pa cm, and the substrate forms a tilt angle greater than 60° with respect to the target.

In a particular embodiment, the process is characterised in that the vacuum achieved is lower than 10⁻⁴ Pa and the chamber fulfils the condition that the L/d quotient be greater than 3.5, where d is the diameter of the target and L is the target-substrate distance.

The selection of the GLAD deposition conditions is critical, since the same nanostructures are not formed under different conditions. The glancing-angle cathode sputtering technique allows for the formation of nanocolumns whose dimensions and spacing depend upon the operating pressure in the chamber, the inclination of the substrate with respect to the flow of atoms originating from the target, the geometry of the deposition system, the substrate and the duration of the deposition.

When working at low pressures, the titanium atoms that form the nanostructured material reach the surface with high energy, causing the nanocolumnar structures to grow with a high aspect ratio and reducing the effective horizontal surface, such that bacteria have a smaller anchoring surface area. Similarly, the value of the L/d quotient ensures that the beam originating from the target which forms the nanostructured titanium is sufficiently collimated to form the nanocolumns.

The titanium targets used in cathode sputtering usually have a high purity, generally greater than 99%, since it makes no sense to work in a vacuum reactor if the target has impurities for most applications.

Cathode sputtering may generate electromagnetic excitation by means that comprise at least one of the following: DC (continuous excitation), RF (alternating excitation within the radiofrequency range) or pulsed DC (excitation with continuous current pulses).

The deposition of the coating on the substrates results in a surface topography formed by nanocolumns. The nanostructured titanium grows during deposition on the surface of the substrate, covering the surface with nanocolumns that have a high degree of density, i.e. a high degree of nanomotifs per unit surface area, with a mean spacing between the nanocolumns of 100 nm.

The implant formed presents a number of advantages with respect to the prior art: the proposed technique (glancing-angle cathode sputtering at low pressures) is more efficient than other techniques, such as anodisation, not only from an energetic standpoint, since it is performed at room temperature and does not generate residues, but also because it allows for a more precise control of the morphology of the coating and avoids chemical treatments; moreover, it allows for the coatings to grow on large surfaces with various shapes, and using an industrially scalable process; since it is performed at room temperature, this allows for synthesis on surfaces that can only be processed at low temperatures, such as polymeric materials; it inhibits bacterial adhesion and the formation of bacterial biofilms on the surface, and presents suitable properties for the adhesion, spreading and proliferation of osteoblasts.

The third object of the invention is the use of the implant in osseous implantology, since it allows for simultaneous opposite behaviours towards osteoblasts and bacteria, specifically towards *S. aureus,* which is the main cause of infection in metallic prostheses.

Another aspect of the invention is a treatment method for humans or animals, which comprises the following steps:
- inserting the implant in a human or animal body by means of surgery, preferably by means of orthopaedic, cranial, dental and/or maxillofacial surgery.

In a particular embodiment, the implant may be for temporary use or for permanent use.

### DESCRIPTION OF THE FIGURES:

**Figure 1****:** Images of the implant obtained by means of scanning electron microscopy (the images on the left are cross-sections, those on the right are bird's eye views), which present nanocolumns obtained under different deposition conditions by means of glancing-angle cathode sputtering. A) GLAD angle 70° and operating pressure in the chamber, or argon pressure, 0.15 Pa. B) GLAD angle 80° and argon pressure 0.15 Pa. C) GLAD angle 85° and argon pressure 0.15 Pa. D) GLAD angle 60° and argon pressure 0.5 Pa.
**Figure 2****:** Images of surfaces that do not present nanocolumns, obtained by means of electron microscopy (on the left, cross-sections; on the right, bird's eye views), obtained under different conditions by means of glancing-angle cathode sputtering: A) GLAD angle 60° and argon pressure 0.15 Pa. B) GLAD angle 60° and argon pressure 1 Pa.
**Figure 3****:** X-ray diffraction diagrams of nano-Ti6Al4V (above) and Ti6Al4V (below) samples, acquired by means of grazing incidence (Ω = 0.5°). The stars (*) indicate the diffraction corresponding to the Ti6Al4V alloy. The Miller indices for the rutile phase of TiO₂ are indicated.
**Figure 4****:** Fourier transform infrared (FT-IR) spectrum of nano-Ti6Al4V (below) and Ti6Al4V (above), obtained by means of attenuated total reflectance (ATR).
**Figure 5****:** SEM images of: (A) Ti6Al4V; (B) nano-Ti6Al4V, where the surface marked with an ellipse indicates an estimate of the size of the osteoblast; (C) nano-Ti6Al4V, where the surface marked with a circle indicates an estimate of the size of *S*. *aureus;* (D) SEM image of a cross-section of nano-Ti6Al4V, showing the nanocolumns.
**Figure 6****:** AFM images of: A) Ti6Al4V, and B) nano-Ti6Al4V. The greyscale on the right indicates the height of the motifs, which has a maximum of 46 nm and 380 nm for Ti6Al4V and for nano-Ti6Al4V, respectively.
**Figure 7****:** Evaluation of the surface wettability: A) Photograph of a drop of water on a Ti6Al4V sample; (B) image of a drop of water on a nano-Ti6Al4V sample; (C) evolution of the contact angle as a function of time for both samples.
**Figure 8****:** (A) Adhesion of the osteoblasts after 90 minutes on nano-Ti6Al4V and Ti6Al4V samples. (B) Mitochondrial activity (MTT assay) after three days of culture on Ti6Al4V and nano-Ti6Al4V.
**Figure 9****:** SEM images obtained after 24 hours of culture with osteoblastic cells on a substrate of (A) and (C), Ti6Al4V; and (B) and (D), nano-Ti6Al4V. In C), some of the anchors formed by the cells are indicated by means of ellipses.
**Figure 10****:** Count of *S. aureus* colonies formed after 90 minutes of culture on nano-Ti6Al4V and Ti6Al4V surfaces. The * indicates statistically significant differences, p < 0.05.
**Figure 11****:** Confocal fluorescence microscopy images after 90 minutes of culture with live and dead *S. aureus* bacteria, (A) and (B), Ti6Al4V; (C) and (D), nano-Ti6Al4V.
**Figure 12****:** SEM images of samples of (A) Ti6Al4V and (B) nano-Ti6Al4V after 24 hours of culture with *S. aureus.* The inset in (a) is the surface of a Ti6Al4V sample prior to the culture.

### EMBODIMENTS OF THE INVENTION

### Example 1: Implant obtained by coating deposition using glancing-angle cathode sputtering on a biomaterial.

In this example, we indicate how the implant was formed.

Using glancing-angle cathode sputtering, a coating was deposited which was formed by nanostructured titanium on a mechanically mirror-polished Ti6Al4V alloy disc (root mean square roughness lower than 5 nm measured on a surface area of 4 µm²), 1 cm in diameter and 2 mm thick. The chamber had a base pressure (prior to the introduction of the gas) lower than 5 × 10⁻⁷ Pa (ultra-high vacuum) and the target-substrate distance was 22 cm. The 5-cm-diameter, 5-mm-thick target used was made of titanium with a purity of 99.999 %, and, on the upper part, had a cylindrical chimney 5 cm in diameter and 9 cm in length (this chimney primarily serves to prevent cross-contamination with other targets in the chamber, but, moreover, contributes to the collimation of the atomic flow, by directing the flow of material towards the surface of the substrate). The L/d parameter had a value of 4.4. During the deposition, the pressure in the reactor, or operating pressure in the chamber, was given by an argon gas pressure ranging between 0.15 and 3 Pa, and the DC excitation had a constant power of 300 W. The temperature of the substrate was maintained below 350 K. The tilt angle ranged between 0° and 85°. The process was performed under the ballistic regime, fulfilling the condition that p_{d}L be lower than 12 in all cases.

The implant obtained was observed using SEM; in Table 1, we may observe when the nanocolumns are formed as a function of the operating pressure in the chamber, which is caused by the inert gas introduced, and the tilt angle of the substrate with respect to the vertical of the substrate:

**Table 1: List of coatings obtained by means of glancing-angle cathode sputtering.**

| | **Tilt angle** | | | | | |
|---|---|---|---|---|---|---|
| **P (Pa)** | 0° | 45° | 60° | 70° | 80° | 85° |
| **0.15** | X | X | X | C | C | C |
| **0.5** | X | X | C | | | |
| **1** | X | X | X | | | |
| **1.5** | X | X | X | | | |
| **3** | X | | | | | |

The cells containing the letter C indicate those situations wherein nanocolumns were observed, and the letter X indicates those situations wherein nanocolumns were not formed.

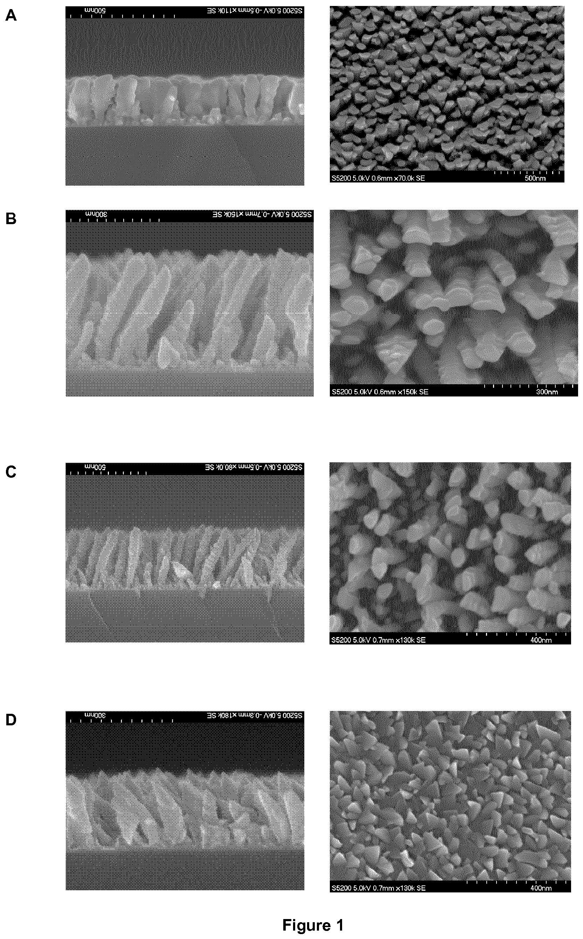

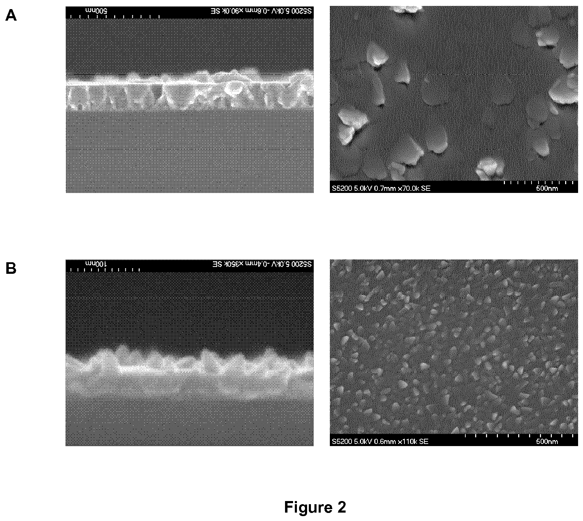

Figure 1 shows several representative cases of nanocolumns observed by means of Scanning Electron Microscopy, or SEM, whereas Figure 2 shows cases wherein the nanocolumns were not formed.

In the case of the obtainment of nanostructured titanium in nanocolumnar form, the nanocolumns obtained have a diameter ranging between 30 and 100 nm, a separation ranging between 50 and 150 nanometres, and an inclination with respect to the vertical of the substrate ranging between 0° and 30°.

### Example 2: Use of the implant in osseous implantology.

In this example, we show that the implant obtained under the conditions of Example 1 have osseointegrative properties that inhibit the formation of a bacterial biofilm.

The implant was obtained following the process of Example 1, using an argon pressure of 0.15 Pa and a GLAD angle of 80°. The temperature of the substrate was maintained below 350 K.

In this particular case, the surface of the implant is the surface of the coating, and is formed by nanostructured titanium which forms nanocolumns with dimensions ranging between 100 and 300 nm in height, and between 30 and 100 nm in diameter. The nanocolumns grow during deposition on the Ti6Al4V surface, and cover the surface with a high degree of density, i.e. a high degree of nanomotifs per unit surface area, with a mean space of 100 nm.

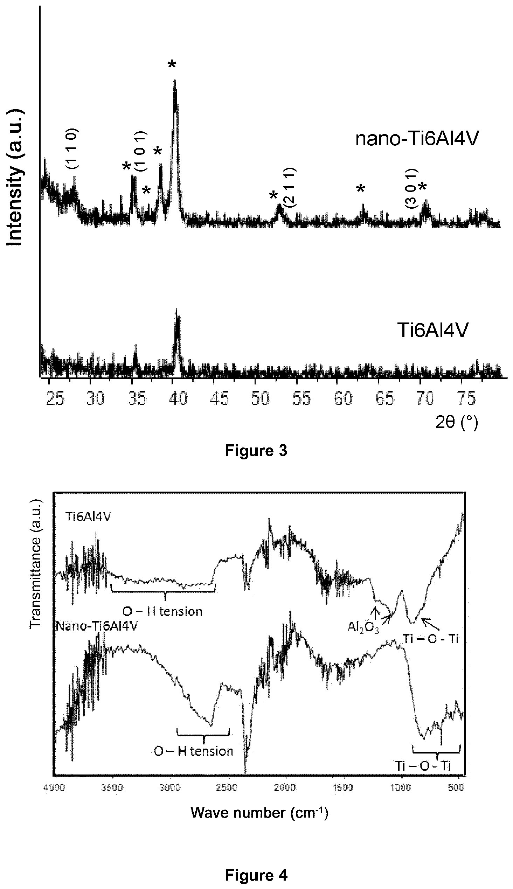

X-ray diffraction studies were performed (represented as X-ray diagrams or XRD) using a Philips X'Pert Model diffractometer in the 2θ range of 20-80. In order to obtain information, preferably about the surface of the disc, the grazing incidence method was used, with a grazing angle ω of 0.5°. Figure 3 shows the X-ray diffraction diagrams obtained by means of grazing incidence for a commercial Ti6Al4V substrate (hereinafter Ti6Al4V) without the coating and for the coating of the invention, or nano-Ti6Al4V. The diffraction maxima of Ti6Al4V may be assigned to the hexagonal phase α-Ti (the main phase of Ti6Al4V alloys) with a P63/mmc space group. The X-ray diffraction diagram for nano-Ti6Al4V shows the diffraction maxima pertaining to the α-Ti phase, jointly with a secondary rutile TiO₂ phase with a P42/mm space group. Fourier transform infrared (FT-IR) spectra were obtained using a Thermo Nicolet Nexus spectrophotometer equipped with a Goldengate attenuated total reflectance (ATR) device. Figure 4 shows the spectrum of Ti6Al4V and nano-Ti6Al4V, and in both samples we may observe absorption bands corresponding to Ti-O-Ti bonds within a wide range of frequencies, between 950 and 500 cm⁻¹, which is indicative of the TiO₂ layers on the surface of Ti6Al4V and nano-Ti6Al4V. Moreover, bands corresponding to the tension mode of the O-H bond are observed, which may be assigned to the presence of Ti-OH groups on the surface.

Finally, phonon mode bands (between 1100 and 1400 cm⁻¹) of Al₂O₃ are observed on the substrate; their presence is characteristic of the surface of the Ti6Al4V alloy. This band does not appear in the nano-Ti6Al4V material, which indicates that the substrate has been effectively coated with the titanium nanocolumns. The presence of diffraction maxima in the X-ray diagram corresponding to a rutile-type TiO₂ phase in nano-Ti6Al4V and the presence of absorption bands in the infrared spectrum attributable to Ti-O-Ti bonds demonstrate the presence of a TiO₂ layer that would be coated with the Ti nanocolumns grown on the Ti6Al4V substrate.

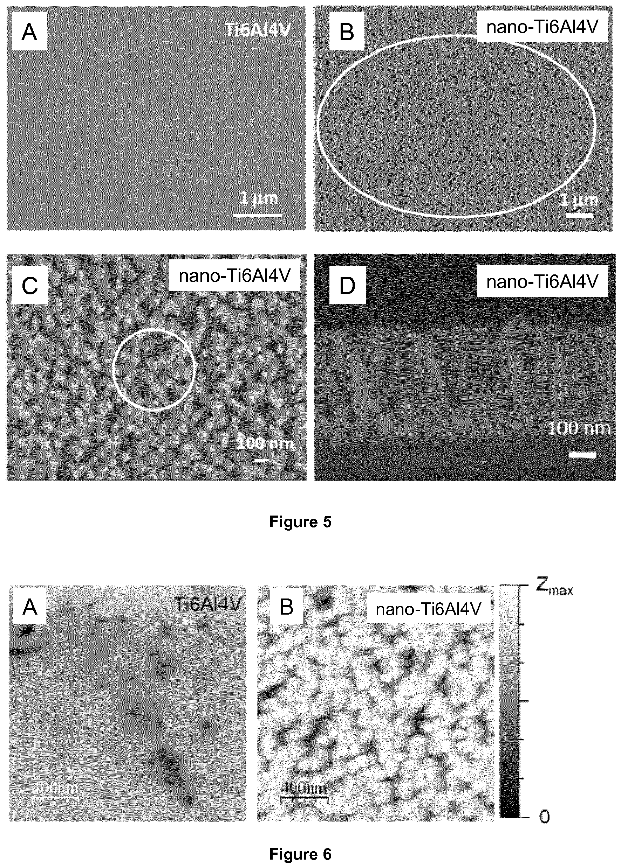

The structure of the implant may be seen in Figure 5. To this end, measurements were taken using SEM. The initial surface of Ti6Al4V does not present any roughness perceptible by SEM (Figure 5A), which corresponds to a mirror-polished surface. However, at the same scale, the nano-Ti6Al4V surface appears to be completely covered by nanoroughness as a result of the deposition of Ti on the Ti6Al4V substrate, due to the growth of nanocolumns. The Atomic Force Microscopy, or AFM, measurements for both surfaces (Figure 6) show the difference in roughness; for Ti6Al4V, it was 3 nm (root mean square value, or RMS) on a surface area of 4 µm², whereas, for nano-Ti6Al4V, the measured roughness value was 57 nm on a surface area of 4 µm².

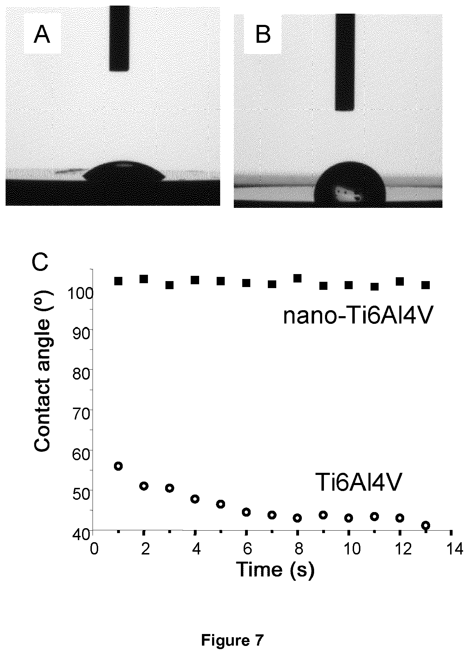

The contact angle was measured by means of the sessile drop method, in a CAM 200 KSV contact angle equipment at 25°C, taking photographs every 1 second. The contact angle studies (Figure 7) indicate a significant increase in hydrophobicity following the coating process. The initial contact angles, after 1 second, were 56° and 102° for Ti6Al4V and for nano-Ti6Al4V, respectively. The contact angle for nano-Ti6Al4V remained constant with time, which indicates low wettability, indicative of hydrophobic surfaces, whereas the contact angle for Ti6Al4V decreased to 44° during the first 8 seconds, which is indicative of the high wettability characteristic of Ti6Al4V alloys.

### Culture of osteoblasts

Prior to the *in vitro* culture of osteoblasts, the samples were sterilised and dried at 150°C for 12 h. A human osteosarcoma (HOS) cell line was used, obtained through the European Collection of Cell Cultures (ECACC, no. 87070202). The cells were cultured in complete medium, composed of Dulbecco's modified Eagle medium (DMEM) (Sigma Chemical Co., St. Louis, USA) supplemented with 2 mM L-glutamine (Gibco, Invitrogen Corporation, USA), 100 U ml⁻¹ penicillin (Life Technologies Limited, Scotland), 100 g ml⁻¹ streptomycin (Life Technologies Limited, Scotland) and 10% foetal bovine serum (FBS) (Gibco, Invitrogen Corporation, USA), at 37°C in a humid atmosphere containing 95% air and 5% CO₂. The HOS cells were routinely trypsinised and subcultured. Subsequently, the HOS cells were seeded in different 24-well plates with a seeding density of 2.5 x 10⁵ cells per ml in complete medium, under a CO₂ atmosphere (5%) at 37°C, for different periods of time for each of the assays.

### Statistics

The data obtained from the osteoblast and bacterial cultures are expressed as the mean ± standard deviation of experiments performed on three different samples. The statistical analysis was performed using the Statistical Package for the Social Sciences (SPSS) software, version 11.5. The statistical comparisons were performed by means of analysis of variance (ANOVA). The differences between the groups were determined by means of post-hoc evaluation using Scheffe's test. For all the statistical evaluations, a difference value was considered to be statistically significant for p < 0.05.

### Cell adhesion of osteoblasts

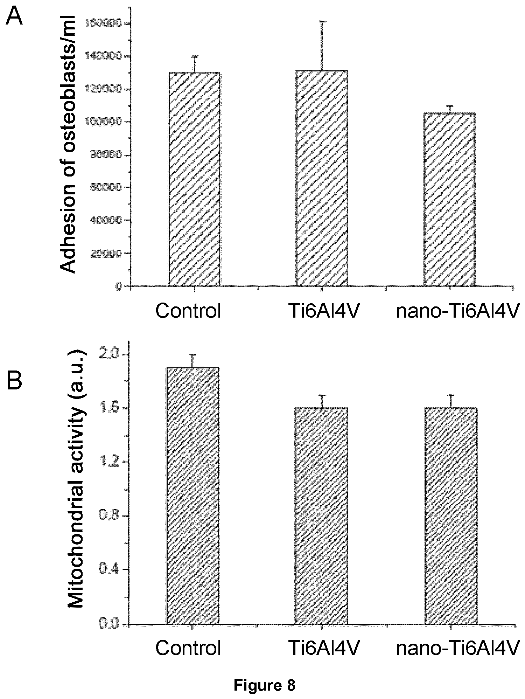

In order to study the adhesion of osteoblasts on the surface of the implant, i.e., in this case, the surface of the coating, the samples were incubated under standard culture conditions for 90 min. Subsequently, the samples were washed three times with PBS; thereafter, the cells were separated by means of a trypsin treatment for 10 min. Following centrifugation, the cells were resuspended in PBS and counted in a Neubauer chamber. Figure 8 indicates the in vitro biocompatibility results performed with HOS on the surface of the coating. To this end, the results of the initial adhesion (90 minutes) and the proliferation of the HOS cells following 3 days of culture were considered, through the quantification of the mitochondrial activity. The data of Figures 8A and 8B are expressed as the mean values ± standard deviation of measurements taken on three different samples. The initial adhesion of the osteoblasts (90 minutes) does not show significant differences between TI6Al4V, nano-Ti6Al4V, and the control (plastic of the culture plate).

### Cell proliferation of osteoblasts

The cell proliferation was determined on the basis of the cellular mitochondrial activity. To this end, the HOS cells were seeded on the surface of the material in 24-well plates, with a density of 10⁵ cells per ml in complete medium, and incubated under standard conditions. The cell proliferation was determined using the MTT assay (3-[4,5-dimethylthiazol-2-yl]-2,5-diphenyltetrazolium bromide) (Sigma-Aldrich, USA) for different periods of time following the seeding. The plastic of the culture plate was used as a control. The quantitative determination was performed in a UV-VIS spectrophotometer, taking a reading at 570 nm. The mitochondrial activity is directly related to the absorbance at said wavelength. The mitochondrial activity of HOS was almost identical for both surfaces and did not show differences with respect to the control following 3 days of culture, as may be observed in Figure 8B.

### Osteoblastic cell stretch assays

The degree of cell stretch and the morphology of the osteoblasts were observed by means of SEM microscopy. The adhered cells were washed three times in PBS and fixed with 2.5% glutaraldehyde (50% wt., Sigma-Aldrich, USA) in PBS for 45 min. The samples were dehydrated by slowly replacing the medium, using ethanol series with an increasing concentration (30%, 50%, 70%, 90%), for 30 min, with a final dehydration in absolute ethanol for 60 min, which allowed for drying of the samples at room temperature under vacuum. The Ti6Al4V and nano-Ti6Al4V samples were mounted on specimen holders and coated with gold for viewing in the SEM.

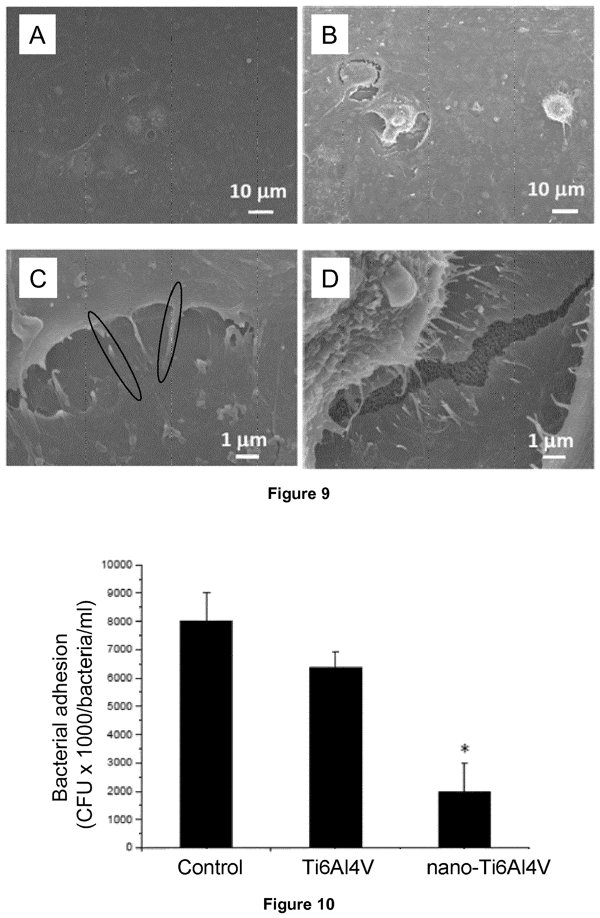

Figure 9 shows the surface following one day of culture of the HOS cells on the surface of the coating. The surface appears to be completely covered by the cells and shows good adhesion, proliferation and degree of stretching. The micrographs obtained at higher magnifications show the anchor elements formed by the cells. Figure 9d shows a detailed view of the nanocolumns beneath the osteoblast layer.

### Bacterial cultures with S. aureus

The preliminary in vitro studies of bacterial adhesion were performed using an ATCC 29213 strain of *Staphylococcus aureus* (*S. aureus*) as a bacterial model under the static conditions commonly specified in the literature (Montanaro L., et al., Future Microbiology 2011, 6 (11): 1329-49). The samples were sterilised by means of dry heat at 150°C for 12 h. The S. *aureus* bacteria grew to their mean logarithmic phase in Todd Hewitt (THB) growth medium (Sigma-Aldrich, USA) at 37 °C, under magnetic stirring at 100 rpm, until the optical density measured at 600 nm reached 1.0. At this point, the cultured bacteria were harvested by means of centrifugation at 1500 rpm for 10 min at room temperature. They were washed 3 times with sterile PBS, maintaining the pH at 7.4, and resuspended in PBS at a concentration of 6 x 10⁸ cells.ml⁻¹. Subsequently, they were incubated at 37°C under magnetic stirring at 100 rpm, for different incubation times, in the presence of the biomaterials under study.

### Adhesion studies for S. aureus

The incubation time for the suspended bacteria was 90 minutes. Subsequently, the samples were aseptically removed from the bacterial suspension and rinsed three times in PBS in order to eliminate the free bacteria. The bacteria bound to the surface of the nanostructured material were quantified by means of the following method: each sample was placed in an Eppendorf tube containing 1 ml of sterile PBS. Thereafter, it was sonicated for 30 s, assuming that 99.9% of the remaining bacteria were separated from the surface. Subsequently, 100 ml of each of the products obtained following the sonication were taken, cultured on Tryptic Soy Agar (TSA) plates (Sigma Aldrich, USA) and incubated overnight at 37° C. The number of colony-forming units (CFU) resulting from the sum of the three sonication processes made it possible to determine the number of original bacteria adhered to the samples. The bacterial cultures of S. *aureus* grown on the Ti6Al4V surfaces (Figure 10) did not show significant differences with respect to the control following 90 minutes of exposure. However, in the case of nano-Ti6Al4V, the adhesion of S. *aureus* was three times lower than that of Ti6Al4V.

### Confocal microscopy of S. aureus

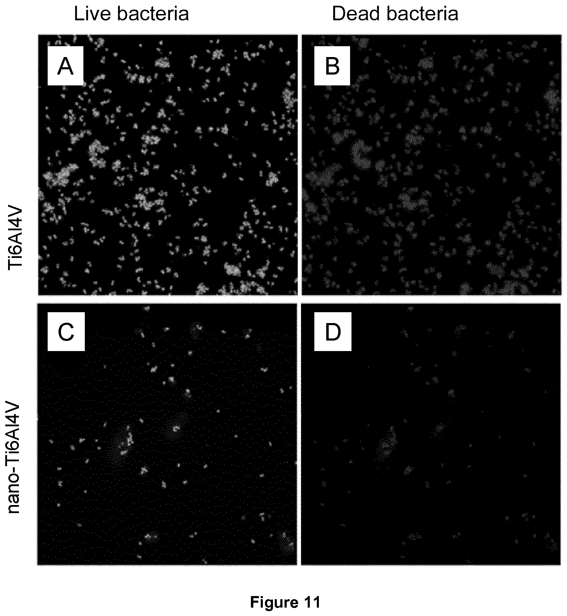

Following 90 minutes of incubation in PBS, the samples were stained for 15 minutes using the Invitrogen Live/Dead BacLight bacterial viability kit. The confocal microscopy studies were performed using a Biorad MC1025 microscope. The SYTO 9 fluorescence (live bacteria, green) is excited at a wavelength of 480/500 nm, and emits fluorescence at 500 nm. The propidium iodide fluorescence (dead bacteria, red) is excited at 490/635 nm, and the fluorescence emitted nm was measured at 618. Figure 11 shows the images obtained by means of confocal microscopy following 90 minutes of culture. The images show less bacterial adhesion on nano-Ti6Al4V, in total agreement with the count shown in Figure 11. No significant differences were observed in the live/dead ratio when the Ti6Al4V and the nano-Ti6Al4V surfaces were compared. This fact suggests that the antibacterial activity of the coatings is exerted thanks to the anti-adhesion properties thereof, without any bactericidal effects against S. *aureus* being observed.

### SEM microscopy of S. aureus

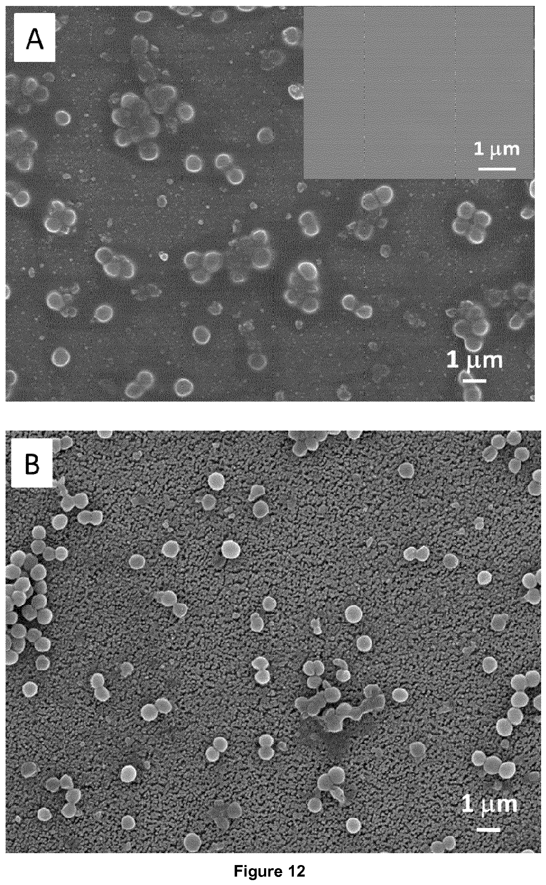

The SEM study was performed by preparing the samples in a manner analogous to that described for the studies with osteoblasts. In Figure 12, we may observe the Ti6Al4V and nano-Ti6Al4V surfaces following 24 hours of culture with S. *aureus.* Figure 12A corresponds to the surface of the Ti6Al4V sample and shows the bacteria surrounded by an extracellular matrix identified as a bacterial biofilm, which covers the polished surface of the substrate. In order to highlight the presence of the bacterial biofilm, Figure 12 contains an inset which shows the clean biofilm surface prior to the bacterial culture, whereas, on the contrary, the surface of the nano-Ti6Al4V sample shows a micrography wherein the bacteria that are present have not been able to form a biofilm, which makes it possible to view the nanostructure of the nano-Ti6Al4V sample.

## Claims

1. Implant that comprises a titanium coating deposited on a substrate, **characterised in that**:
- the substrate comprises a biomaterial with a root mean square roughness lower than 5 nm on a surface area of 4 µm²,
- the coating has a purity greater than 95% and comprises nanostructured titanium formed by metallic titanium and a titanium oxide layer,
- the nanostructured titanium has a nanocolumnar form, wherein the diameter of the nanocolumns ranges between 30 and 100 nm, the height ranges between 100 and 300 nanometres, and the space between the nanocolumns ranges between 50 and 150 nanometres, with a nanocolumn tilt angle with respect to the vertical of the substrate ranging between 0° and 30°.

2. Implant according to claim 1, wherein the substrate biomaterial comprises at least one of the following materials:
- commercially-pure medical-grade titanium with a purity greater than 99%, for periodontal implants,
- medical-grade metallic alloys, such as CrCo, stainless steel and Ti6Al4V, for orthopaedic, cranial and maxillofacial applications.

3. Implant according to claim 2, wherein the substrate biomaterial comprises Ti6Al4V.

4. Implant according to any of claims 2 to 3, wherein the substrate biomaterial is shaped into structures that comprise discs, bolts, nails, rods, osteosynthesis plates and other fracture fixation devices, generally manufactured with stainless steel.

5. Process for obtaining the implant of claims 1 to 4, which comprises depositing the coating on the substrate using glancing-angle (GLAD) techniques.

6. Process according to claim 5, wherein the deposition is performed in a cathode sputtering system.

7. Process according to claim 6, wherein the cathode sputtering system comprises a magnetron.

8. Process according to claim 7, wherein the deposition comprises the following steps:
a) introduction of the substrate into the cathode sputtering system chamber,
b) closing of the chamber and creation of a vacuum,
c) introduction of gas into the chamber,
d) electromagnetic excitation of the gas particles present in the chamber by means of a source,
e) collision of the particles present in the chamber against a titanium target,
f) deposition of the material detached from the target on the substrate,
wherein the product of multiplying the operating pressure (P_{g}) by the target-substrate distance (L) fulfils the ballistic regime condition for the sputtering of Ti, given by p_{g}L < 12 Pa cm, and the substrate forms a tilt angle greater than 60° with respect to the target.

9. Process according to claim 8, wherein the vacuum reached is lower than 10⁻⁴ Pa, and the chamber fulfils the condition that the L/d quotient is greater than 3.5, where d is the diameter of the target and L is the target-substrate distance.

10. Use of the implant defined in claims 1 to 4 in osseous implantology.

11. Use according to claim 10, in implants for temporary use or for permanent use.
